# EUROPÄISCHE PATENTSCHRIFT

(11) **EP 3 369 368 B1**
(45) Veröffentlichungstag und Bekanntmachung des Hinweises auf die Patenterteilung: **05.02.2020**
(21) Anmeldenummer: 18020052.9
(22) Anmeldetag: 08.02.2018
(51) Int. Cl.: A61B 5/00, A61B 5/042, A61B 18/14

(54) **MULTI-ELEKTRODEN ANORDNUNG**
MULTI-ELECTRODE ASSEMBLY
DISPOSITIF À ÉLECTRODES MULTIPLES

(30) Priorität: 01.03.2017 DE 102017001971
(43) Veröffentlichungstag der Anmeldung: 05.09.2018
(73) Patentinhaber: Peter Osypka Stiftung, 79639 Grenzach-Wyhlen (DE)
(72) Erfinder: OSYPKA, Peter, 79639 Grenzach-Wyhlen (DE)

(56) Entgegenhaltungen:
- EP-A1- 2 682 157
- EP-A1- 3 023 052
- EP-A1- 3 111 872
- US-A- 5 558 073
- US-A- 5 876 336
- US-A1- 2006 106 375

## Beschreibung

Die Erfindung betrifft einen Mapping Katheter mit einer Multi Elektroden Anordnung (Elektroden Array), der Areale des Herzens, insbesondere die Vorhöfe hochauflösend abtastet (mapped), um die Ausbreitung der elektrophysiologischen Signale bei Vorhofflimmern zu messen, durch elektrische Stimulation zu unterdrücken oder durch Hochfrequenz-Ablation zu beseitigen. Ferner erlaubt die Multi Elektroden Anordnung auch das Beseitigen des Vorhofflimmerns durch Kardioversion.

Vorhofflimmern ist eine Störung, bei der elektrische Störsignale einen unregelmäßigen Herzschlag verursachen. Insgesamt scheint Vorhofflimmern in den letzten Jahren zugenommen zu haben, was mit einem immer älter werdenden Patientenkollektiv erklärt wird. Vorhofflimmern führt zu einer schnellen Überleitung der Erregung auf die Ventrikel, so dass es zu akuter hämodynamischer Instabilität kommen kann. Die Beseitigung von Vorhofflimmern durch Hochfrequenz Ablation erfolgt bisher durch einen im linken Vorhof eingeführten Katheter, der nach der sogenannten "Mace Prozedur" neben den Ausgängen der Pulmonal Venen auch andere Teile im linken Vorhof abladiert. Die Ausbreitung der elektrophysiologischen Signale im linken Vorhof ist noch nicht genau erforscht und von Patient zu Patient unterschiedlich. Um die Belastung des Patienten möglichst gering zu halten, bedarf es an mehr physiologischer Informationen im linken Vorhof. Vor Beginn der Ablation sollte Information über die individuelle Anatomie der Vorhöfe vorliegen.

Die Europäische Patentanmeldung EP3023052 (Biosense Webster) beschreibt einen Elektroden Array bestehend aus einer Vielzahl von langestreckten Bandelementen (Spines) mit einer Vielzahl von Ringelektroden, die auf dem nicht-leitenden Überzug der langestreckten Bandelemente aufgebracht sind. Die Bandelemente verlaufen im distalen Bereich parallel zueinander. Die distalen Enden sind frei oder können als loop ausgebildet sein. Die Elektrodendichte beträgt circa 15 Elektroden pro cm2.

Die Europäische Patentanmeldung EP3111872 (Biosense Webster) beschreibt einen Elektroden Array mit geschlossenen Schleifen, wobei jede Schleife eine Vielzahl von Ringelektroden trägt. Die Elektroden tragenden Abschnitte verlaufen parallel zueinander. Die Elektrodendichte beträgt circa 15 Elektroden pro cm2.

Das US Patent US5876336 beschreibt einen Elektroden Array in Basket Struktur mit flexiblen Bandelementen (Spines), die die Elektroden tragen. Wie die Elektroden ausgebildet sind wird nicht näher erläutert. Der Hinweis auf einen Zuleitungsdraht, der zu jedem Pol führt weist aber auf eine Art Ringelektrode hin.

Die US Patentanmeldung US20060106375 beschreibt einen Elektroden Array ebenfalls in einer Art Basket Struktur. Die Elektoden sind auf einem Isolator aufgebracht, was einer Ringelektrode entspricht.

Das US Patent US5558073 beschreibt einen Elektroden Array in Basket Struktur mit flexiblen Bandelementen (spaced apart arms).Elektroden sind auf dem Bandelement aufgebracht, was einer Ringelektrode entspricht. EP-A-2 682 157 beschreibt eine Elektroden-Anordnung, die aus einem geflochtenen Schirm aus Memory Metall Drähten besteht.

Die Aufgabe wird gelöst durch einen Mapping Katheter mit einer Multi-Elektroden Anordnung nach dem vorliegenden Anspruch 1.

Das Array eignet sich zur Aufnahme von elektrischen Signalen (Mapping), zum Stimulieren und zur Beseitigung von Vorhofflimmern durch Abladieren. Das Array eignet sich auch zur Kardioversion.

Das Shape Memory Metall ist beispielsweise Nitinol. Wenn im Folgenden Nitinoldraht erwähnt wird, sind auch immer andere Shape Memory Metalle gemeint.

Der Sensing-Bereich ist der Teil der Wendel, der Pole aufweist. Die Länge des Sensing-Bereichs kann variieren, Sie soll 5-20mm betragen, beispielsweise circa 15 mm. Die Länge des Sensing-Bereichs kann pro Wendel gleich sein oder auch variieren.

Der Ausdruck eine "Vielzahl von dicht nebeneinander angeordneten Polen" bedeutet z.B 16 Pole, die maximal vorhanden sein können wenn 16 Drähte zu einer Wendel gewickelt werden. Die nebeneinander liegenden Pole bilden ein "Sensing Paket". Bei einem Drahtdurchmesser von 0.12mm und 16 Drähten betragt der mit Polen versehene Abschnitt , (Sensing Paket) (16 x 0.12mm), d.h. circa 2 mm. Eine Länge des Sensing Bereichs von 5-20mm wird durch Änderung der Wendelsteigung erreicht. Durch Änderung der Wendelsteigung lässt sich auch der Abstand der Pole steuern.

Der Ausdruck "dicht nebeneinander angeordnet" bedeutet, dass der Abstand zwischen zwei Polen klein ist, kleiner 5mm, kleiner 2mm oder kleiner 1mm, beispielsweise 0,2 mm oder kleiner 0,2mm, beispielsweise 0,08- 0,2mm.

Mehr als 16 Pole pro Wendel können erreicht werden durch Veränderung der Wickeltechnik sodass mehr als 16 Drähte zu einer Wendel gewickelt werden können oder durch Ineinanderschieben von Wendeln mit unterschiedlichem Durchmesser. Das Ineinanderschieben von 2 Wendeln mit je 16 Polen könnte 32 Pole ergeben, das Ineinanderschieben von 3 Wendeln mit je 16 Polen könnte 48 Pole ergeben usw. Es ist auch möglich Wendeln mit einzelnen Polen mit blanken Wendeln zu kombinieren. Durch die Kombination ergeben sich neben der Möglichkeit zum Mapping auch Möglichkeiten zum Ablatieren oder Kardiovertieren.

Das Elektroden Array besteht aus einer Vielzahl von Wendeln. Eine Vielzahl von Wendeln bedeutet mehr als eine Wendel, mehr als 2 Wendeln, beispielsweise 4 oder mehr als 4 Wendeln, beispielsweise 4-16 Wendeln.

Die Anzahl der Pole im gesamten Elektroden Array hängt von den vorhandenen Polen pro Wendel und von der Anzahl der Wendeln ab. Beides kann variieren, sodass eine besonders hohe Flexibilität und Varianz in der Polanordnung und Polanzahl gegeben ist.

Besteht das Elektrodenarray beispielsweise aus 8 Wendeln mit 16 Polen pro Wendel erreicht man 128 Pole, bei 12 Wendeln sind es 192 Pole, usw.

Der Aufbau des Elektroden Arrays führt dazu, dass in Gebrauchsstellung alle Pole des Arrays einen engen Kontakt zum Endokard, beispielsweise zur Vorhofwand oder zu den Pulmonal Venen und Pulmonalvenenostien haben, sodass bei einem einzigen Mapping-Vorgang eine Vielzahl von elektrischen Signalen simultan gemessen wird und somit der zeitliche Ablauf der Potentialverteilung aufgezeichnet werden kann (zuverlässiges Sensing). Somit kann für jeden Patienten eine individuelle "Landkarte" der elektrophysiologischen Signale im Herzen erstellt werden. Der gute Kontakt der Multipol Wendeln zum Endokard wird durch die vorgeformten Nitinoldrähte in den Wendeln erreicht.

In einer Ausführungsform eignet sich die erfindungsgemäße Multi-Elektroden Anordnung zur Aufzeichnung von elektrischen Signalen in den Pulmonal Venen und im Bereich der Pulmonalvenenostien. Die Multipol-Wendeln sind um eine Längsachse gruppiert. In den Multipol-Wendeln verlaufen vorgeformte Nitinoldrähte die bedingen, dass die Wendeln nach Expansion des Elektroden Arrays an der Wand der Pulmonal Venen und am Pulmonalvenenostium anliegen oder angedrückt werden können.

In einer Ausführungsform eignet sich die erfindungsgemäße Multi-Elektroden Anordnung zur Aufzeichnung von elektrischen Signalen im rechten und/oder linken Vorhof. Die Multipol-Wendeln sind um eine Längsachse gruppiert. Entlang der Längsachse verläuft vorzugsweise ein Führungsdraht um den sich die Multipolwendeln gruppieren. In den Multipol-Wendeln verlaufen vorgeformte Nitinoldrähte die bedingen, dass die Wendeln nach Expansion des Elektroden Arrays an der Vorhofwand anliegen oder angedrückt werden können. Der vorgeformte Nitinoldraht ist in der Wendel frei beweglich und weist beispielsweise einen Durchmesser von 0,1-0,5 mm auf. Der vorgeformte Nitinoldraht wird über das distale Ende der Wendel hinaus weitergeführt.

In einer Ausführungsform werden die Enden der vorgeformten Nitinoldrähte, die in jeder Wendel verlaufen, alle miteinander verbunden. Die Verbindung der vorgeformten Nitinoldrähte führt zu einer verbesserten Stabilität des Sensing Bereichs und verhindert ein Verrutschen der kreisförmig gruppierten Multipolwendeln.

In einer Ausführungsform ist der Führungsdraht am distalen Ende gerade geformt. Die distalen Enden der vorgeformten Nitinoldrähte, die in jeder Wendel verlaufen, werden alle miteinander verbunden und zusätzlich mit dem zentralen Führungsdraht verbunden. Diese Anordnung führt neben der oben erwähnten verbesserten Stabilität auch zu der Möglichkeit über Schieben oder Ziehen am zentralen Führungsdraht den Kontakt der Pole zum Endokard zu steuern. Der Sensing Bereich kann konvex oder konkav ausgerichtet werden. So kann das Mapping Areal entsprechend der Anatomie des Vorhofs konkav bzw. konvex angepasst werden, was zu einem besonders engen Kontakt der Pole zum Endokard führt und zu einer zuverlässigen Aufzeichnung der Signale.

In einer Ausführungsform ist der zentral verlaufende Führungsdraht an seinem distalen Ende kreisförmig und die Nitinoldrähte, die in den Wendeln verlaufen werden an dem kreisförmigen Ende befestigt. Auch in dieser Ausführungsform kann durch Druck oder Zug am Führungsdraht eine konvexe oder konkave Ausrichtung des Sensing Bereichs erreicht werden.

Durch Schieben oder Ziehen am Führungsdraht, der mit den Nitinoldrähten in den Wendeln und somit indirekt mit den Wendeln verbunden ist, lässt sich der Druck der Wendeln gegen die Vorhofwand und somit der Kontakt der Pole einstellen oder Nachjustieren. Wichtig ist ein enger Kontakt der Pole zum Endokard.

Der Führungsdraht ist vorzugsweise auch aus Nitinol, kann aber auch aus einem anderen Metall wie beispielsweise aus Edelstahl sein.

Benachbarte Wendeln haben entweder denselben Abstand zueinander, sodass ein regelmäßiges Gruppieren gegeben ist oder die Gruppierung ist unregelmäßig, das heißt die Abstände zwischen je zwei benachbarten Wendeln variieren.

Der Führungsdraht kann gegebenenfalls Träger von weiteren Polen, von Sensoren und/oder von Hilfsmitteln sein, wie beispielsweise Träger von LED -Vorrichtungen.

Das Areal des Vorhofs, welches mit dem Elektroden Array in einem Mapping Vorgang simultan abgetastet werden kann, bildet das Mapping-Areal. Planare, konvexe oder konkave Areale können abgetastet werden. Durch Vorschieben des Führungsdrahts wölbt sich das Array nach außen (konvex), durch Zug nach innen (konkav).

Die Multipol-Wendel wird aus einer Vielzahl von parallel verlaufenden isolierten Drähten gefertigt und die Pole werden durch punktförmiges Entfernen der Isolationsschicht auf dem einzelnen Draht gebildet. Die Wendel muss weich, flexibel und dennoch stabil sein.

Durch die Wahl des Drahtmaterials der Wendel, durch den Drahtdurchmesser und durch den Abstand der einzelnen Windungen (Wendelsteigung) können die Eigenschaften der Wendel bezüglich Flexibilität, Elastizität und Stabilität beeinflusst und vorgewählt werden.

Der Drahtdurchmesser der Wendeln beträgt beispielsweise 0,08-0,20 mm, insbesondere 0,08mm; 0,1mm; 0,12mm; 0,15mm; 0,20mm. Dieser Drahtdurchmesser Bereich gewährleistet eine weiche elastische Wendel, die aufgrund ihrer Weichheit und Elastizität einen verbesserten Kontakt zur Vorhofwand ermöglicht.

Der Wendeldurchmesser beträgt beispielsweise 0,4mm - 2,0mm, vorzugsweise 0,6-0,8mm.

Die eingesetzten Wendeln können unterschiedliche Durchmesser haben. So ist es möglich einen Sensing Bereich zu erhalten, der aus ineinandergeschobenen Wendeln besteht, wodurch die Anzahl Pole pro Wendel erhöht werden kann.

Geeignetes Drahtmaterial sind alle biokompatiblen elektrisch leitenden Drähte. Die Drähte der Wendel sind beispielsweise aus Edelstahl, Elgiloy (Co-Cr-Ni Alloy) MP35N(Ni-Co - Legierung), Isotan (CuNi44), Nitinol, Platin, Pt-Ir, Wolfram, Cu, Cu-Pt.

Die Isolation der Drähte besteht beispielsweise aus Polyimid, Polyurethan, Silikon, Pebax, PTFE, Polyamid oder anderen biokompatiblen Kunststoffen.

Die Pole bilden blanke Stellen des Wendeldrahts. Es können aber auch Metallbeaufschlagungen, beispielsweise Goldbeaufschlagungen (Goldbumps) vorgesehen sein. Auf der Oberfläche der Drähte werden Teile abisoliert und mit einer Goldoberfläche versehen. Die Goldbeaufschlagungen haben z.B. einen Durchmesser von 0,1-0,2 mm.

Jede Wendel weist eine Vielzahl von Polen auf. Die Pole sind entweder in einer Linie angeordnet oder leicht versetzt in Zick-Zack-Form. Die Anzahl der Pole und die Anzahl der parallel verlaufenden Drähte (Windungen) können variieren. Wichtig für die Anwendung ist ein geringer Abstand der Pole um eine hohe Mappingdichte zu ermöglichen. Durch leichtes Versetzen der Pole auf der Wendel (Zick-zack) erlangt man sichere und kurze Abstände der Pole.

Zur Reduzierung der Zuleitungsdrähte zu den Polen können Multiplexer eingesetzt werden.

Der besondere Vorteil eines derartigen Elektroden Arrays ist es, dass man im Array (und damit im Herzen) bestimmte Areale hochauflösend selektieren kann, wodurch der Mapping Vorgang beschleunigt wird.

Mit Hilfe der Pole werden elektrische Signale des Endokards aufgezeichnet um den Ursprung des Vorhofflimmerns zu orten. Da eine hohe Anzahl von Polen vorhanden ist und die Poldichte hoch ist kann das Vorhofareal durch simultanes Mapping vollkommen und hochauflösend abgetastet werden. Ist ein Vorhofareal gefunden, welches Ursprung der Signalstörung sein könnte, kann dieses Areal quasi "rausvergrößert" werden und noch genauer abgetastet werden. Dabei ist die Varianz in der Polanordnung hilfreich.

Die Signalaufzeichnung mit Hilfe des erfindungsgemäßen Elektroden Arrays lässt sich auch in einem Computer gesteuerten Verfahren anwenden oder in einem durch Ultraschall geführten Mappingverfahren.

Ist der Ursprung des Vorhofflimmerns geortet, kann mit derselben Elektrodenanordnung die Ablation vorgenommen werden. Zum Mappen und nachfolgendem Abladieren eignet sich eine Ausführungsform bei der zwischen jedem isolierten Draht der Wendel ein blanker Draht parallel dazu liegt über den Hochfrequenzstrom (RF-Strom) abgegeben wird. Als blanker Draht kann Drahtmaterial wie oben für Wendeldraht beschrieben verwendet werden.

Mit Hilfe der Pole können nicht nur elektrische Signale im Vorhof aufgenommen werden (Sensing), sondern ebenso Areale im Vorhof stimuliert werden. Durch Anlegen eines elektrischen Impulses zwischen zwei Polen kann eine Stimulation erfolgen.

Selbst die Kardioversion ist möglich. Hierzu eignet sich eine Elektrodenvorrichtung wie sie zum Abladieren zum Einsatz kommt. Blanke Drähte zwischen den isolierten Drähten der Wendel stellen den indifferenten Pol beim Kardioversions Prozess dar.

Zur Vergrößerung der Polfläche für die Kardioversion können zwischen den Multipol-Wendeln blanke Wendeln angeordnet sein.

Das Kernstück der vorliegenden erfindungsgemäßen Elektrodenanordnung ist die hohe Anzahl von Polen pro Wendel, die zu einer hohen Mappingdichte führt sowie das Vorhandensein von vorgeformten Drähten aus Memory Metall in den Wendeln, was zu einem guten Kontakt der Pole mit dem Endokard führt. Im Katheter Schaft ist die Form noch unterdrückt, nach Ausschieben des Elektroden Arrays kommt die vorgegebene Form selbstständig zur Wirkung (selbstexpandierend).

Die Vorformung der Drähte kann Patienten spezifisch auf Basis von digitalen Aufnahmen der Vorhöfe geschehen. Die digitalen Aufnahmen können aus medizinischen Aufnahmen wie beispielsweise MRT-, CT-, Ultraschall-, oder Röntgenaufnahmen erhalten werden.

In einer Ausführungsform hat der Katheter Schaft des Katheters ein Lumen zur Einführung des Elektroden Arrays.

In einer weiteren Ausführungsform hat der Katheter Schaft des Katheters mehr als ein Lumen, beispielsweise zwei Lumen. Durch das zweite Lumen kann ein Gegendruckelement geführt werden, welches in Gebrauchsstellung dem Elektroden Array gegenüberliegt.

Das Gegendruckelement bewirkt je nach Anatomie des Vorhofs einen verbesserten Kontakt der Pole zum Endokard. Gegendruckelemente sind beispielsweise Nitinolgeflechte oder Ballons. Auch durch eine Schraubwendel oder durch einen Abstandhalter kann ein Gegendruck erzeugt werden.

Die erfindungsgemäße Multi-Elektrodenanordnung eignet sich zum Abtasten des rechten und des linken Vorhofs. Zur Einführung der Multi-Elektrodenanordnung in den linken Vorhof ist eine Punktion des Vorhof-Septums notwendig. Eine geeignete Vorrichtung zur Punktion des Vorhof-Septums ist beispielsweise in EP2674189 beschrieben.

### Bezugszeichenliste

- 1: Katheter Schaft
- 2: Elektroden Array
- 3: Multipol Wendel
- 4: Nitinoldraht in der Wendel
- 5: Zuleitungsbereich der Wendel
- 6: Führungsdraht mit kreisförmigem distalem Ende
- 7: Sensing-Bereich der Wendel
- 8: Abtastareal (Mapping Areal)
- 9: Führungsdraht
- 10: Pol
- 11: Blanker Draht
- 12: Nitinolgeflecht als Gegendruckelement
- 13: Ballon als Gegendruckelement
- 14: Ballon als Gegendruckelement
- 15: Abstandshalter als Gegendruckelement
- 16: Schraubwendel als Gegendruckelement
- 17: Steuerbarer Katheter
- 18: Vorhofareal

- 20: Pulmonal Vene
- 21: Pulmonalvenenostium
- 22: Vorhof-Septum

- 30: Einführungsvorrichtung

Die folgenden Figuren erläutern die Erfindung.

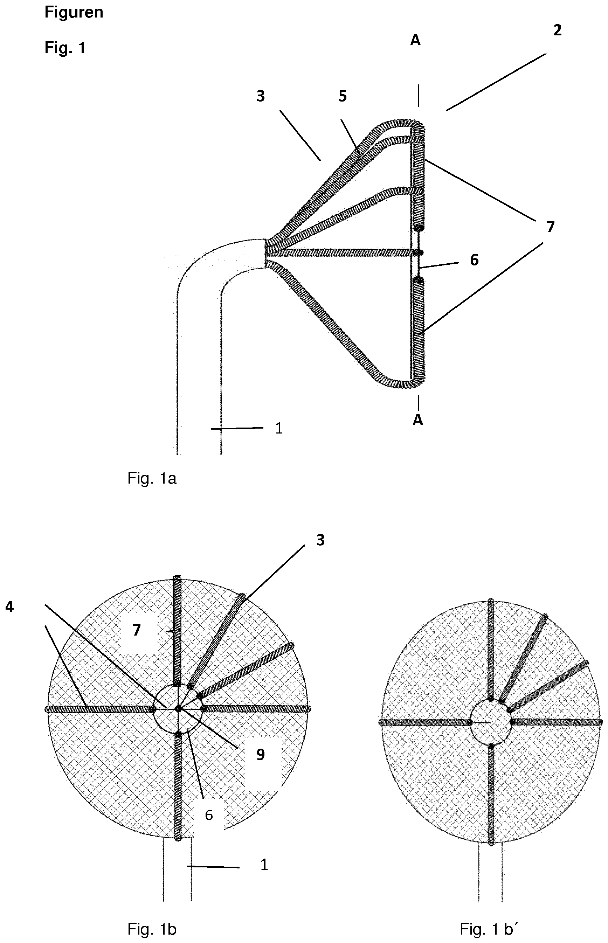

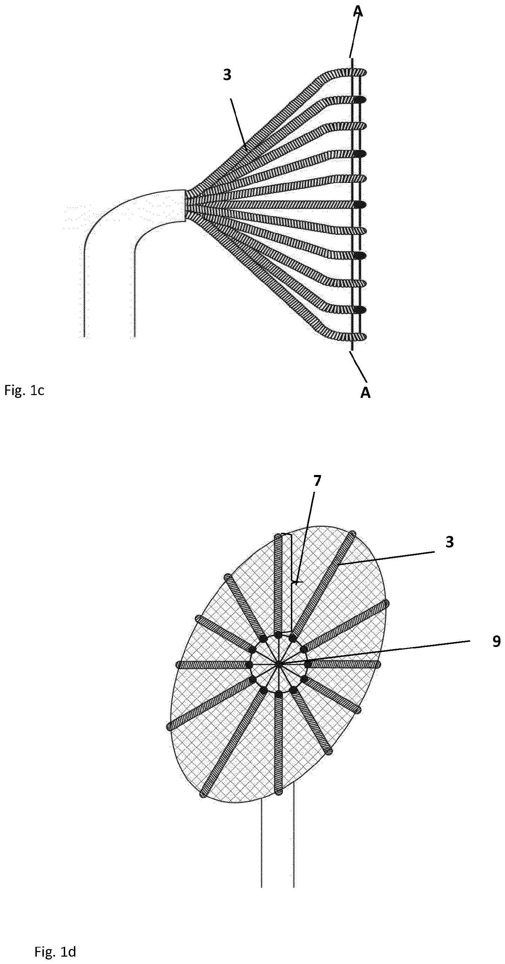

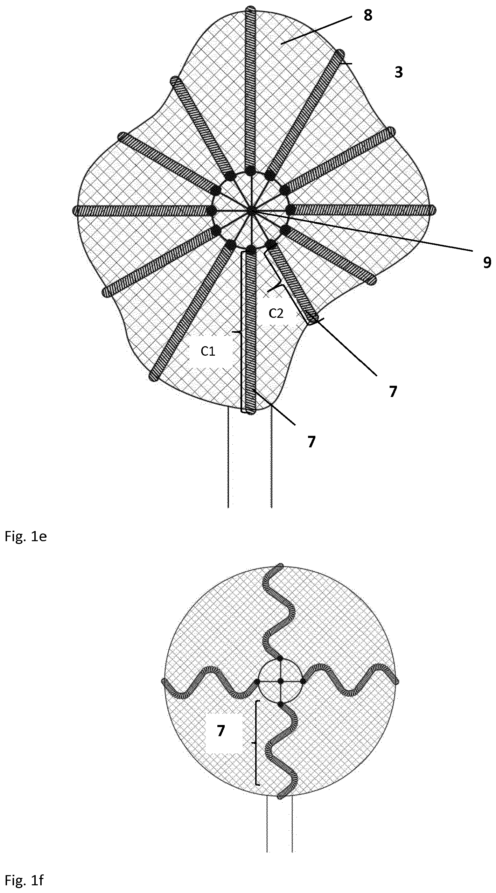

**Fig. 1** zeigt die erfindungsgemäße Elektrodenanordnung schematisch in Seitenansicht (Fig. 1a, 1c) und in Aufsicht (Fig. 1b, 1d, 1e, 1f,)
   Fig. 1a zeigt den Katheter Schaft (1) des Katheters und das Elektroden Array (2), welches sich am distalen Ende des Katheter Schafts befindet. Das Elektroden Array ist im expandierten Zustand. Er besteht aus den Multipol Wendeln (3), die distal aus dem Katheter Schaft herausragen und einen Zuleitungsbereich (5) und einen Sensing-Bereich (7) aufweisen. Ein Führungsdraht (6) verläuft innerhalb des Schafts und entlang einer Längsachse im Zentrum des Arrays und ist entweder gerade oder an seinem distalen Ende kreisförmig gebogen. Die Multipolwendeln gruppieren sich kreisförmig um den Führungsdraht. In den Wendeln verlaufen vorgeformte Nitinoldrähte (4)(in der Seitenansicht nicht dargestellt), die mit dem Führungsdraht (6) verbunden sind und die bedingen, dass die Sensing-Bereiche (7) in Gebrauchsstellung einen engen Kontakt zum Endokard haben.
   Fig. 1b ist eine Aufsicht entlang der Linie A-A von Fig. 1a. Die Multipol Wendeln (3) sind kreisförmig angeordnet und weisen Sensing-Bereiche (7) auf. Der vorgeformte Nitinoldraht (4), der durch die Wendeln verläuft, ist über das distale Ende der Wendeln hinausgeführt und mit dem Führungsdraht (9) verbunden. In der Aufsicht ist der zentral verlaufende Führungsdraht (9) als Punkt sichtbar. 6 Wendeln sind vorhanden. Der Abstand von je zwei benachbarten Wendeln variiert.
   Der Kreis (6) deutet die weitere Ausführungsform an, wobei die Wendeln mit einem Führungsdraht verbunden sind, der an seinem distalen Ende kreisförmig gebogen ist..
   Fig. 1c ist eine Seitenansicht entsprechend der Figur 1a. Im Unterschied zur Fig. 1a sind mehr Wendeln (3) vorhanden.
   Fig. 1d ist eine Aufsicht entlang der Linie A-A in Fig. 1c. Die Sensing-Bereiche (7) umspannen ein elliptisches Areal. 12 Wendeln sind um den Führungsdraht (9) gruppiert.
   Fig. 1e zeigt eine Elektrodenanordnung wobei das Mapping Areal (8) den anatomischen Verhältnissen des Herzens des Patienten angepasst wurde. (custom made). Die Länge des Sensing-Bereichs (7) ist für jede Wendel (3) unterschiedlich (c1, c2, bis c12). Alle Nitinoldrähte, die in den Wendeln verlaufen, sind distal mit dem Führungsdraht (9) verbunden.
   Fig. 1f zeigt einen Array entsprechend Fig. 1b. Im Unterschied zu Fig. 1b wird die Länge des Sensing-Bereichs (7) durch wellenförmige Vorformung des in der Wendel verlaufenden Nitinoldrahts vergrößert. Eine derartige Verlängerung des Sensing-bereichs erlaubt eine Varianz in der Position der Pole. Eine Kombination von wellenförmig angeordneten Wendeln und gerade verlaufenden Wendeln ist auch möglich.

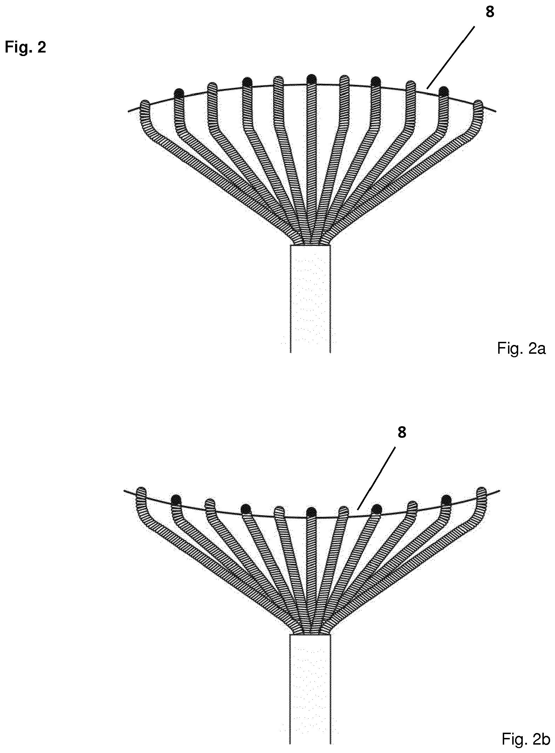

**Fig. 2** zeigt die erfindungsgemäße Elektrodenanordnung schematisch in Seitenansicht entsprechend Fig. 1c. Im Gegensatz zu Fig. 1c zeigt Fig. 2 einen konvexen (Fig. 2a) oder konkaven(Fig. 2b) Verlauf. Dadurch dass die Nitinoldrähte, die in jeder Wendel verlaufen mit dem Führungsdraht verbunden sind, kann über Druck oder Zug am zentralen Führungsdraht die konvexe bzw. konkave Ausrichtung erreicht werden. So kann das Mapping Areal (8) entsprechend der Anatomie des Vorhofs konkav bzw. konvex angepasst werden.

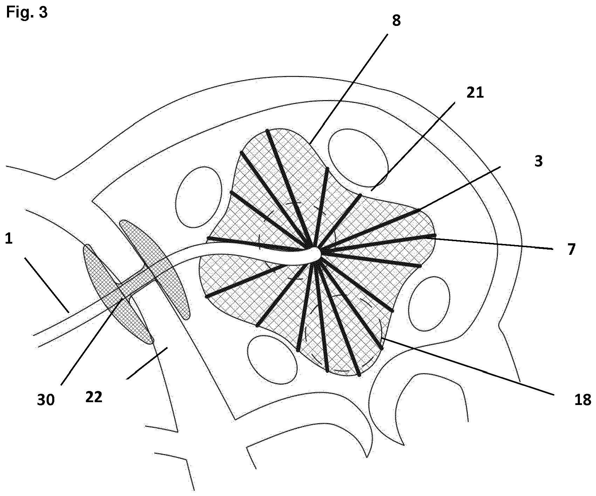

**Fig. 3** zeigt die Elektrodenanordnung gemäß Fig. 1e im linken Vorhof positioniert. Der Sensing Bereich (7) und somit das Mapping Areal (8) ist Patienten spezifisch geformt. Bei Bedarf können einzelne Vorhofareale (18) detaillierter abgetastet werden. Die Areale (18) sind durch eine gestrichelte Linie angedeutet. Signale im Bereich des Pulmonalvenenostiums (21) können auch aufgezeichnet werden. Der enge Kontakt zum Endokard kann wie oben beschrieben über Druck oder Zug am zentralen Führungsdraht erreicht werden.
   Der Katheter Schaft (1) des Katheters ist durch das Vorhof Septum (22) geführt mit Hilfe einer Vorrichtung (30) beispielsweise einer Vorrichtung gemäß EP 2674189. Die Vorrichtung besteht aus einem Nitinolgeflecht mit einer Öffnung und einem Ventil, die den Zugang zum linken Vorhof ermöglicht.
   Mit dieser Vorrichtung ist auch ein Multisite Pacing möglich, wobei das Nitinolgeflecht der Vorrichtung (30) die großflächige indifferente Elektrode darstellt und die Pole der Wendeln (3) den differenten Pol darstellen.

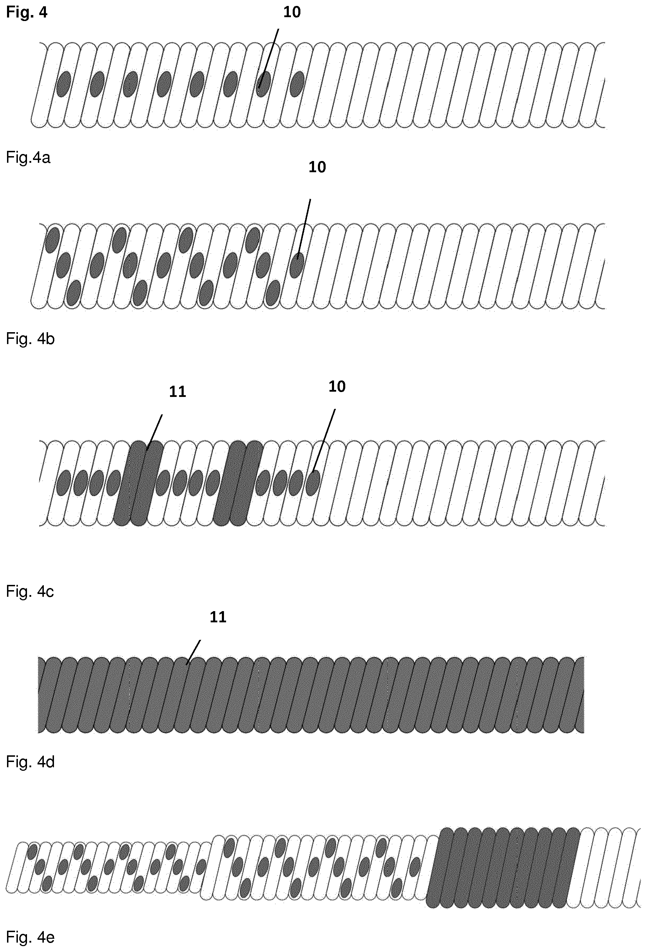

**Fig. 4** zeigt die Wendeln.
   Fig. 4a ist eine Wendel aus isoliertem Draht mit 16 parallel verlaufenden Drähten. Die Pole (10) sind durch Entfernen der Isolationsschicht entstanden und verlaufen in einer Linie. Die in Fig. 4a dargestellte Wendel eignet sich zum Aufzeichnen von elektrischen Signalen und zum Stimulieren.
   Die in Fig. 4b dargestellte Wendel eignet sich zum Aufzeichnen von elektrischen Signalen und zum Stimulieren. Die Pole (10) verlaufen versetzt (zick zack förmig). Durch das leichte Versetzen der Pole auf der Wendel erlangt man sichere und kurze Abstände der Pole. So können 16 Pole dicht nebeneinander platziert werden.
   Fig. 4c zeigt eine Wendel, die sich zum Abladieren und zum Kardiovertieren eignet. Blanke Drähte (11) liegt zwischen den isolierten Drähten, die die Pole (10) tragen.
   Fig. 4d zeigt eine blanke Wendel, die im Array zwischen den Multipol Wendeln angeordnet werden kann.
   Fig. 4e zeigt eine Wendelkombination aus 3 Wendeln, zwei Wendeln wie in Fig. 4b dargestellt und eine blanke Wendel. Die Wendeln haben unterschiedlichen Durchmesser und lassen sich so ineinander stecken.

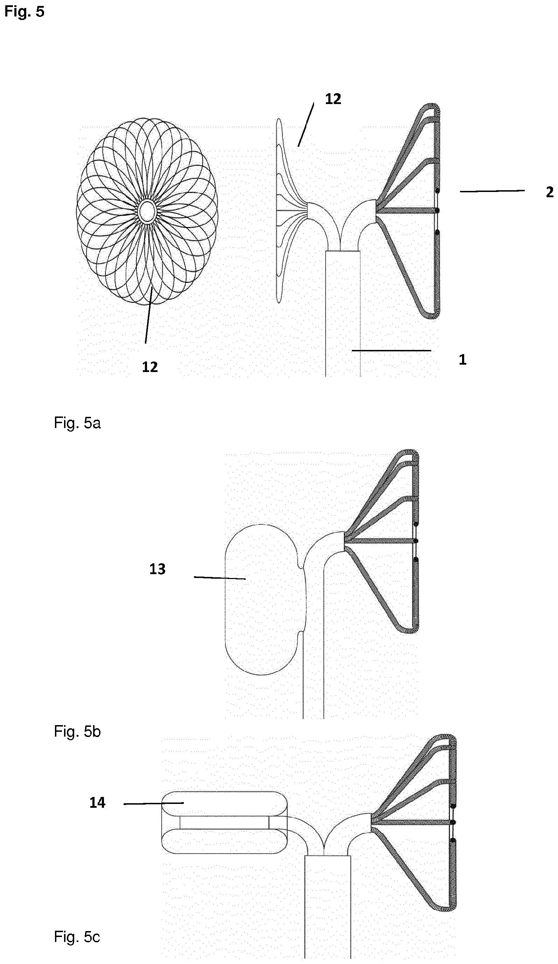

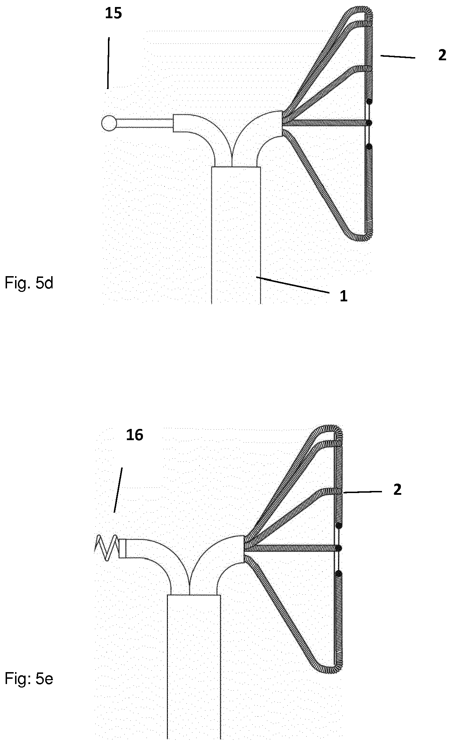

**Fig. 5** zeigt einen Katheter mit Gegendruckelement.
   Fig. 5a zeigt einen zweilumigen Katheter Schaft (1) wobei durch das zweite Lumen ein Nitinolgeflecht (12) als Gegendruckelement geführt ist. Das Elektroden Array (2) entspricht dem in Fig. 1a dargestellten Elektroden Array.
   Fig. 5b zeigt einen Ballon (13) als Gegendruckelement.
   Fig. 5c zeigt ebenfalls einen Ballon(14) als Gegendruckelement.
   Fig. 5d zeigt einen Abstandshalter (15) als Gegendruckelement.
   Fig. 5e zeigt eine Schraubwendel (16) als Gegendruckelement

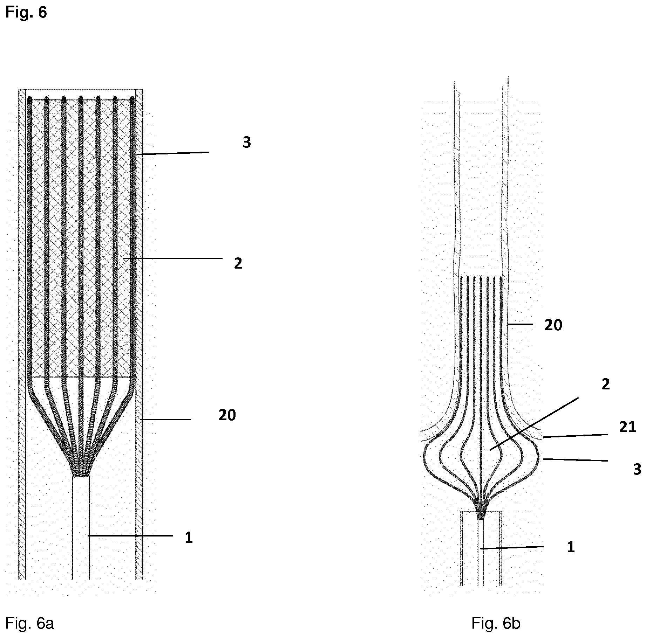

**Fig. 6** zeigt das Abtasten der Pulmonal Venen (20) schematisch in Seitenansicht.
   Fig.6a zeigt den Katheter Schaft (1) des Katheters und das Elektroden Array (2), das sich am distalen Ende des Katheter Schafts befindet. Das Elektroden Array ist im expandierten Zustand. Das Array besteht aus den Multipol Wendeln (3), die distal aus dem Katheter Schaft herausragen. In den Wendeln verlaufen vorgeformte Nitinoldrähte (in der Fig. nicht sichtbar), die bedingen, dass die Wendeln an der Wand der Pulmonal Venen (20) anliegen.
   Fig. 6b zeigt das Abtasten des Pulmonalvenenostiums (21) schematisch in Seitenansicht.

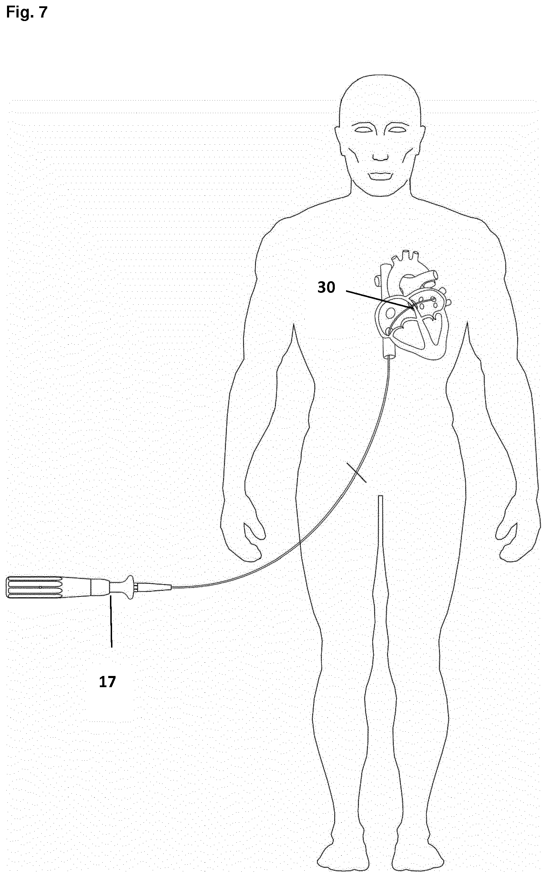

**Fig. 7** zeigt die gesamte Katheter Vorrichtung in Gebrauchsstellung. Der steuerbare Katheter (17) wird über die untere Vene in den rechten Vorhof geführt und mit Hilfe der Vorrichtung (30) durch das Septum in den linken Vorhof bzw. in die Pulmonal Venen.

## Patentansprüche

1. Multi-Elektroden Anordnung bestehend aus
einem Katheter dessen Katheter Schaft (1) mindestens ein Lumen aufweist und ein distales und ein proximales Ende hat; und aus
einem Elektroden Array (2), wobei das Elektroden Array (2) komprimierbar und selbstexpandierbar ist und sich im expandierten Zustand am distalen Ende des Katheter Schafts (1) befindet, und wobei das Elektroden Array (2) eine Vielzahl von Multipol-Wendeln (3) mit Polen (10) aufweist, wobei die Wendeln aus einer Vielzahl von parallel verlaufenden gewickelten isolierten Drähten (Windungen) besteht, und die Pole (10) blanke Stellen der Isolationsschicht darstellen, die Multipol-Wendeln (3) sind um eine Längsachse gruppiert und wobei jede Multipol-Wendel einen Zuleitungsbereich (5) und einen Sensing-Bereich (7) aufweist,
**dadurch gekennzeichnet dass** der Sensing-Bereich (7) jeder Wendel eine Vielzahl von dicht nebeneinander angeordneten Polen (10) aufweist, wobei jede Wendel ein Lumen aufweist, wobei durch das Lumen der Wendeln je ein vorgeformter Draht aus Shape Memory Metall (4) geführt ist, wobei der vorgeformte Draht ein distales und ein proximales Ende hat, und wobei das Elektroden Array (2) im expandierten Zustand eine Form annimmt, die durch die vorgeformten Drähte (4) aus Shape Memory Metall in den Wendeln definiert wird, sodass in Gebrauchsstellung die Pole (10) engen Kontakt zum Endokard haben.

2. Multi-Elektroden Anordnung nach Anspruch 1, wobei das Shape Memory Metall Nitinol ist.

3. Multi-Elektroden Anordnung nach Anspruch 1, wobei entlang der Längsachse ein Führungsdraht (6) mit kreisförmigem distalen Ende oder ein Führungsdraht (9) mit geradem distalen Ende (9) verläuft um den sich die Multipol-Wendeln (3) gruppieren.

4. Multipolanordnung nach Anspruch 3, wobei die vorgeformten Nitinoldrähte, die in jeder Wendel verlaufen über das distale Ende der Wendeln hinausgeführt werden, miteinander an ihren distalen Enden verbunden sind und mit dem Führungsdraht (9) verbunden sind.

5. Multipolanordnung nach Anspruch 3, wobei die vorgeformten Nitinoldrähte, die in jeder Wendel verlaufen mit dem kreisförmigen distalen Ende von Führungsdraht (6) verbunden sind.

6. Multi-Elektroden Anordnung nach Anspruch 1, wobei die Wendel (3) aus einer Vielzahl von parallel verlaufenden gewickelten isolierten Drähten (Windungen) gefertigt ist und die Pole (10) durch punktförmiges Entfernen der Isolationsschicht gebildet werden.

7. Multi-Elektroden Anordnung nach Anspruch 1, wobei 16 Pole pro Wendel vorhanden sind.

8. Multi-Elektroden Anordnung nach Anspruch 1, wobei 2 oder mehr Wendeln ineinandergesteckt und somit kombiniert werden.

9. Multi-Elektroden Anordnung nach Anspruch 8, wobei zwei Wendeln mit je 16 Polen und eine blanke Wendel kombiniert werden.

10. Multi-Elektroden Anordnung nach Anspruch 1, wobei der Drahtdurchmesser der Wendeln (3) 0,08-0,20 mm beträgt, insbesondere 0,08mm; 0,1mm; 0,12mm; 0,15mm; 0,20mm.

11. Multi-Elektroden Anordnung nach Anspruch 1, wobei 4-16 Wendeln vorhanden sind.

12. Multi-Elektroden Anordnung nach Anspruch 1, wobei der Führungsdraht (6) oder (9) Träger einer LED Vorrichtung ist.

13. Multi-Elektroden Anordnung nach Anspruch 1, wobei der Führungsdraht (6) oder (9) Träger von Sensoren ist.

14. Multi-Elektroden Anordnung nach Anspruch 1, wobei der Führungsdraht (6) oder (9) Träger von Ultraschall-Sensoren ist.

15. Multi-Elektroden Anordnung nach Anspruch 1, wobei der Katheter Schaft (1) zwei Lumen aufweist und durch das zweite Lumen ein Gegendruckelement geführt ist

## Claims

1. Multi-pole assembly consisting of
a catheter having a catheter shaft (1); the catheter shaft (1) has at least one lumen and has a distal and a proximal end; and consisting of
an electrode array (2) being self-expandable from a collapsed structure to an expanded structure and being arranged at the distal end of the catheter shaft (1) when expanded,
and wherein the electrode array (2) comprises a plurality of multipole coils (3) with poles (10) wherein each multipole coil (3) is made of a plurality of insulated coiled wires running in parallel and the poles (10) are bare areas of the insulating layer and are formed by spot-removing the insulating layer;
the multipole coils (3) are grouped around the longitudinal axis of the assembly and each multipole coil has an electrical supply region (5) and a sensing region (7), **characterized in that** the sensing region (7) of each coil has a plurality of poles (10) being arranged adjacent to each other; wherein each coil has a lumen through which a preformed wire of shape memory metal (4) is guided, wherein the preformed wire has a distal and a proximal end, and
wherein the expanded electrode array (2) is shaped according to the pre-shaped wire (4) of shape memory metal running inside the coils so that in the position of use the poles (10) have close contact to the endocard tissue.

2. The multi-pole assembly according to claim 1, wherein the shape memory metal is nitinol.

3. The multi-pole assembly according to claim 1, wherein a guide wire (6) having a circular shaped distal end or a guide wire (9) having a straight distal end runs along the longitudinal axis and wherein the multipole coils are grouped around the guide wire (6).

4. The multi-pole assembly according to claim 3, wherein the preformed nitinol wires running through each multipole coil are connected to the distal end of the guide wire (9).

5. The multi-pole assembly according to claim 3, wherein the preformed nitinol wires running through each multipole coil are distally led out of the coil and are interlinked and are connected to the circular distal end of the circular shaped guidewire (6).

6. The multi-pole assembly according to claim 1, wherein each multipole coil (3) is made of a plurality of insulated coiled wires running in parallel and the poles (10) are formed by spot-removing the insulating layer.

7. The multi-pole assembly according to claim 1, wherein there are 16 poles per coil.

8. **The** multi-pole assembly according to claim 1, wherein 2 or more coils are plugged into each other and thus combined.

9. The multi-pole assembly according to claim 8, wherein two coils each having 16 poles and a bare coil are combined.

10. The multi-pole assembly according to claim 1, wherein the wire diameter of the coils (3) is 0.08-0.20 mm, especially 0.08 mm; 0.1mm; 0.12 mm; 0.15mm; 0.20mm.

11. The multi-pole assembly according to claim 1, wherein there are 4-16 coils.

12. The multi-pole assembly according to claim 1, wherein the guide wire (6) or (9) is a support of an LED device.

13. The multi-pole assembly according to claim 1, wherein the guidewire (6) or (9) is a carrier of sensors.

14. The multi-pole assembly according to claim 1, wherein the guide wire (6) or (9) is a carrier of ultrasonic sensors.

15. The multi-pole assembly according to claim 1 wherein the catheter shaft (1) has two lumens and a counter pressure element is passed through the second lumen.

## Revendications

1. Assemblage multipolaire constitué d'un cathéter avec une tige (1); la tige de cathéter (1) a au moins une lumière et possède une extrémité distale et une extrémité proximale; et constitué d'un réseau d'électrodes (2) qui est compressible et auto-expansible et se trouve à l'extrémité distale de la tige de cathéter (1) lorsque le cathéter est à l'état expansé; et le réseau d'électrodes (2) comprend une pluralité de bobines multipolaires (3) avec des pôles(10); chaque bobine multipolaires (3) est constituée d'une pluralité de fils isolés courent en parallèle et les pôles (10) sont des points dénudé sur la couche isolante est sont formés en éliminant par points la couche isolante ;
les bobine multipolaires (3) sont groupées autour d'un axe longitudinal de l'assemblage et chaque bobine multipolaire a une région d'alimentation (5) et une région de détection (7), **caractérisé en ce que** la région de détection (7) de chaque bobine comporte une pluralité de pôles (10) qui sont disposés l'un à côté de l'autre ;
chaque bobine a une lumière, dans lequel est guidé un fil préformé (4) en métal à mémoire de forme; le fil préformé a une extrémité distale et une extrémité proximale, et dans lequel le réseau d'électrodes (2) à l'état expansé adopte une forme définie par les fils préformés (4) en métal à mémoire de forme qui passent dans les bobines, de sorte qu'en position d'utilisation les pôles (10) sont en contact étroit avec le tissue d'endocarde.

2. Assemblage multipolaire selon la revendication 1, dans lequel le métal à mémoire de forme est le nitinol.

3. Assemblage multipolaire selon la revendication 1, dans lequel un fil de guidage passe le long de l'axe longitudinal ; le fil de guidage est circulaire (6) à l'extrémité distale ou le fil de guidage est en ligne droite (9) à extrémité distale; les bobines multipolaires (3) se groupent autour du fil de guidage.

4. Assemblage multipolaire selon la revendication 3, dans lequel les fils de nitinol préformés qui passent dans chaque bobine sont guidés au delà du fin des bobines et sont interconnectés à leurs extrémités distales et sont connectés au fil de guidage (9).

5. Assemblage multipolaire selon la revendication 3, dans lequel les fils de nitinol préformés qui passent dans chaque bobine sont connectés au fil circulaire de guidage (6) à l'extrémité distale.

6. Assemblage multipolaire selon la revendication 1, dans lequel la bobine (3) est constituée d'une pluralité de fils isolés parallèles et les pôles (10) sont formés en éliminant par points la couche isolante.

7. Assemblage à électrodes multiples selon la revendication 1, dans lequel il y a 16 pôles par bobine.

8. Assemblage multipolaire selon la revendication 1, dans lequel deux bobines ou plus sont enfichées l'une dans l'autre et sont ainsi combinées.

9. Assemblage multipolaire selon la revendication 8, dans lequel deux bobines ayant chacune 16 pôles et une bobine nue sont combinées.

10. Assemblage multipolaire selon la revendication 1, dans lequel le diamètre de fil des bobines (3) est compris entre 0,08 et 0,20 mm, en particulier 0,08 mm; 0.1mm; 0,12 mm; 0.15mm; 0.20mm.

11. Assemblage multipolaire selon la revendication 1, dans lequel il y a 4 à 16 bobines.

12. Assemblage multipolaire selon la revendication 1, dans lequel le fil de guidage (6) ou (9) est un support d'un dispositif à LED.

13. Assemblage multipolaire selon la revendication 1, dans lequel le fil de guidage (6) ou (9) est un support de capteurs.

14. Assemblage multipolaire selon la revendication 1, dans lequel le fil de guidage (6) ou (9) est un support de capteurs à ultrasons.

15. Assemblage multipolaire selon la revendication 1, dans lequel la tige de cathéter (1) a deux lumières et un élément de contre-pression passe à travers la deuxième lumière.
